# EUROPEAN PATENT SPECIFICATION

(11) **EP 0 544 115 B1**
(45) Date of publication and mention of the grant of the patent: **07.01.1998**
(21) Application number: 92118411.5
(22) Date of filing: 28.10.1992
(51) Int. Cl.: C07K 1/22

(54) **Method for binding immunoglobulin G to protein A**
Verfahren zur Bindung des Immunoglobulin G an Protein A
Procédé de fixation de l'immunoglobuline G à la protéine A

(30) Priority: 26.11.1991 JP 335531/91
(43) Date of publication of application: 02.06.1993
(73) Proprietor: TOSOH CORPORATION, Yamaguchi-ken 746 (JP)
(72) Inventor: Kakita, Hirotaka, Shinnanyo-shi, Yamaguchi-ken (JP); Komiya, Katsuo, Hikari-shi, Yamaguchi-ken (JP); Nakamura, Koji, Shinnanyo-shi, Yamaguchi-ken (JP); Kato, Yoshio, Shinnanyo-shi, Yamaguchi-ken (JP)
(74) Representative: VOSSIUS & PARTNER

(56) References cited:
- EP-A- 0 391 526
- EP-A- 0 432 629

## Description

The present invention relates to a method for binding immunoglobulin G (hereinafter referred to simply as IgG) and more particularly, to a method of binding IgG to protein A which is immobilized on an insoluble matrix.

Protein A is capable of binding to the Fc portion of IgG and has been employed as a means for separating and purifying IgG, as immobilized on an insoluble matrix.

The binding of protein A and IgG was first discovered by Kronvall et al.(Journal of Immunology, 105, 1116 (1970)).

Subsequently, Mackenzie et al. attempted to improve the separation of IgG using a protein A-Sepharose 4B column. However, the amounts of mouse IgG 1 and IgG 2 thereby bound were small, and the reproducibility was also poor (Journal of Immunology, 120, 1493 (1978)). Bywater et al. studied purification of IgG employing 16 different types of elution buffers, but were unable to improve the conventional methods (Journal of Immunological Methods, 64, 1 (1983)). On the other hand, Hector et al. improved the binding of IgG to protein A immobilized on an insoluble matrix by using a buffer solution having a pH of from 8.5 to 9.5 containing from 1 to 4M of an inorganic salt (US Patent 4,704,366). However, this method had drawbacks such that the concentration of the salt was so high that proteins precipitated, the viscosity of the solution was also high, and particularly in the case of a salt containing halogen ions, corrosion was likely to result at the metal portions of the apparatus for separation and purification.

Further, Ngo et al. proposed to use a buffer solution having a pH of from about 6 to 10 containing at least one polycarboxylic acid in an amount within a range of from about 0.5 to 0.9M, to improve the binding of IgG to protein A immobilized on an insoluble matrix (U.S. Patent 4,933,435). However, this method had the problem that the recovery of IgG was inadequate.

It is the object of the present invention to provide a method of binding IgG to protein A which overcomes the problems of the prior art. The problems are solved by using a solvent having certain specific liquid properties, i.e. a buffer with a specific pH range containing a tartarate in an amount within a specific range, in a method for binding IgG to protein A immobilized on an insoluble matrix.

Namely, the present invention provides a method for binding immunoglobulin G (IgG) to protein A immobilized on an insoluble matrix, wherein a buffer solution having a pH of from 7 to 9.5 containing from 1.0 to 1.6M of a tartarate is used.

Now, the present invention will be described in detail with reference to the preferred embodiments.

As the tartarate to be used in the present invention, any tartarate may be employed so long as it is soluble in water. The concentration of the salt is within a range of from 1.0 to 1.6M, preferably from 1.0 to 1.4M.

The salt employed to obtain a buffer solution having a pH of from 7 to 9.5, may be any salt so long as it has a buffering ability to bring the pH to from 7 to 9.5.

The insoluble matrix may be any matrix such as polyacrylamide, agarose, cellulose, polyhydroxyalkyl methacrylate or silica.

Now, the present invention will be described in further detail with reference to Examples. However, it should be understood that the present invention is by no means restricted to such specific Examples.

### EXAMPLE 1

TSKgel Toyopearl HW type (trade name, manufactured by TOSOH CORPORATION) used in these Examples, is a packing material for medium-speed gel filtration chromatography composed essentially of a hydrophilic vinyl polymer. TSKgel Toyopearl HW-65 (trade name, manufactured by TOSOH CORPORATION) used in these Examples, is a hydrophilic vinyl polymer having a protein exclusion limit molecular weight of about 500,000.

TSKgel Toyopearl HW-65 was activated by cyanogen bromide (CNBr) to obtain CNBr-TSKgel Toyopearl HW-65. Protein A was immobilized to CNBr-TSKgel Toyopearl HW-65 to obtain Protein A-TSKgel Toyopearl HW-65.

1 ml of the above packing material Protein A-TSKgel Toyopearl HW-65 was packed into an open column (inner diameter: 0.8 cm, length: 15 cm) and equilibrated by allowing 5 ml of a buffer solution A (0.1M glycine + 1.2M sodium tartarate, pH:9.0) to flow therethrough three times. 1.5 ml of sheep serum (manufactured by The Binding Site LTD, sold by Seikagaku Kogyo K.K.) or goat serum (manufactured by ICN Immuno Biologicals) was diluted with 1.5 ml of the buffer solution A and added to the column to allow the binding of IgG to protein A.

The column was washed with 10 ml of the buffer solution A. Then, 3 ml of 0.1M glycine-HCl (pH2.8) was passed therethrough to elute and recover IgG, whereby 17.6 mg of IgG was recovered from the sheep serum, or 15.6 mg of IgG was recovered from the goat serum. Each of the recovered IgG was found to be highly pure from the study by SDS (Sodium Lauryl Sulfate)-electrophoresis or by gel filtration chromatography.

### EXAMPLE 2

1 ml of protein A-TSKgel Toyopearl HW-65 was packed into an open column (inner diameter: 0.8 cm, length: 15 cm) and equilibrated by allowing 5 ml of a buffer solution B (buffer solution of pH9.0 containing sodium tartarate and 0.1M glycine) to flow therethrough three times. Then, 2 ml of sheep serum (manufactured by The Binding Site LTD, and sold by Seikagaku Kogyo K.K.) was diluted with 2 ml of the buffer solution B and added to the column to allow the binding of IgG to protein A.

The column was washed with 10 ml of the buffer solution B. Then, 3 ml of 0.1M glycine-HCl (pH2.8) was passed therethrough to elute and recover IgG.

The recovery of IgG from the sheep serum when the concentration of sodium tartarate in the buffer solution B was 0.8M, 1.0M, 1.2M, 1.4M and 1.6M, were 15.2 mg, 16.2 mg, 19.5 mg, 22.5 mg and 26.6 mg, respectively. It was impossible to prepare a buffer solution B containing 1.8M sodium tartarate because of the limitation in the solubility of sodium tartarate.

### EXAMPLE 3

Sepharose 4B (trade name, manufactured by Pharmacia) disclosed in Example 3, is a packing material for low-speed gel filtration chromatography composed essentially of agarose.

Sepharose 4B was activated by cyanogen bromide (CNBr) to obtain CNBr-Sepharose 4B. Protein A was immobilized to CNBr-Sepharose 4B to obtain protein A-Sepharose 4B.

1 ml of Protein A-Sepharose 4B was packed into an open column (inner diameter: 0.8 cm, length: 15 cm) and equilibrated by allowing 5 ml of a buffer solution A (0.1M glycine + 1.2M sodium tartarate, pH:9.0) to flow therethrough three times. Then, 0.5 ml of sheep serum (manufactured by The Biding Site LTD, and sold by Seikagaku Kogyo K.K.) was diluted with 0.5 ml of the buffer solution A and added to the column to allow the binding of IgG to protein A.

The column was washed with 10 ml of the buffer solution A. Then, 3 ml of 0.1M glycine-HCl (pH2.8) was passed therethrough to elute and recover IgG, whereby 5.5 mg of IgG was recovered. The purity of the recovered IgG was found to be high from the study by SDS-electrophoresis or by gel filtration chromatography.

### COMPARATIVE EXAMPLE 1

The operation was conducted in the same manner as in Example 1 except that the buffer solution A in Example 1 was changed to a buffer solution C (0.1M glycine + 0.7M sodium tartarate, pH:9.0), whereby 12.1 mg of IgG was recovered from a sheep serum, and 13.4 mg of IgG was recovered from a goat serum. These recoveries were 69% and 86%, respectively, of the values obtained in Example 1.

### COMPARATIVE EXAMPLE 2

The operation was conducted in the same manner as in Example 2 except that the concentration of sodium tartarate in the buffer solution B in Example 2 was changed to 0.8M and 1.8M, whereby at the sodium tartarate concentration of 0.8M, 15.2 mg of IgG was recovered from a sheep serum, but at the sodium tartarate concentration of 1.8M, it was impossible to prepare such a buffer solution because of the limitation in the solubility of sodium tartarate.

### COMPARATIVE EXAMPLE 3

The operation was conducted in the same manner as in Example 3 except that the buffer solution A in Example 3 was changed to a buffer solution C (0.1M glycine + 0.7M sodium tartarate, pH:9.0), whereby 3.8 mg of IgG was recovered from a sheep serum. This recovery was 69% of the value obtained in Example 3.

As described in the foregoing, according to the method of the present invention, a buffer solution having a pH range of from 7 to 9.5 containing a tartarate within a range of from 1.0 to 1.6M, is employed in a method for binding IgG to protein A immobilized on an insoluble matrix, whereby the recovery of IgG can be improved while maintaining the purity of the separated and purified IgG at a high level, and it is possible to prevent precipitation of a protein or a substantial increase in the viscosity of the solution.

## Claims

1. A method for binding immunoglobulin G (IgG) to protein A immobilized on an insoluble matrix, wherein a buffer solution having a pH of from 7 to 9.5 containing from 1.0 to 1.6M of a tartarate is used.

2. The method according to Claim 1, wherein the buffer solution contains from 1.0 to 1.4M of the tartarate.

3. The method according to Claim 1, wherein the insoluble matrix is polyacrylamide, agarose, cellulose, polyhydroxyalkyl methacrylate or silica.

## Patentansprüche

1. Verfahren zur Bindung von Immunglobulin G (IgG) an Protein A, das an eine unlösliche Matrix gebunden ist, wobei eine Pufferlösung verwendet wird, die einen pH-Wert von 7 bis 9,5 aufweist und ein Tartrat in einer Konzentration von 1,0 bis 1,6 M enthält.

2. Verfahren nach Anspruch 1, wobei die Pufferlösung das Tartrat in einer Konzentration von 1,0 bis 1,4 M enthält.

3. Verfahren nach Anspruch 1, wobei die unlösliche Matrix Polyacrylamid, Agarose, Cellulose, Polyhydroxyalkylmethacrylat oder Siliciumdioxid ist.

## Revendications

1. Procédé de liaison de l'immunoglobuline G (IgG) à la protéine A immobilisée sur une matrice insoluble, dans lequel on utilise une solution tampon ayant un pH de 7 à 9,5 contenant de 1,0 à 1,6 M d'un tartrate.

2. Procédé selon la revendication 1, dans lequel la solution tampon contient de 1,0 à 1,4 M du tartrate.

3. Procédé selon la revendication 1, dans lequel la matrice insoluble est un polyacrylamide, de l'agarose, de la cellulose, du poly(méthacrylate d'hydroxyalkyle) ou de la silice.
